Europäisches Patentamt

(19) European Patent Office

Office européen des brevets

(11) Publication number: **0 136 803 B1**

# EUROPEAN PATENT SPECIFICATION

(45) Date of publication of patent specification: **30.10.91**    (51) Int. Cl.⁵: **C12P 7/18**

(21) Application number: **84305747.2**

(22) Date of filing: **22.08.84**

(54) Industrial-scale process for the production ofpolyols by fermentation of sugars.

(30) Priority: **24.08.83 GB 8322750**

(43) Date of publication of application:
**10.04.85 Bulletin 85/15**

(45) Publication of the grant of the patent:
**30.10.91 Bulletin 91/44**

(84) Designated Contracting States:
**AT BE CH DE FR GB IT LI NL SE**

(56) References cited:
**US-A- 3 947 323**

**APPLIED AND ENVIRONMENTAL MICROBI-
OLOGY, vol. 32, no. 1, July 1976, pages 56-63,
American Society for Microbiology, US; L.
HANSSENS et al.: "Types of respiratory ac-
tivity in Moniliella tomentosa during growth
under different conditions"**

(73) Proprietor: **CPC INTERNATIONAL INC.
International Plaza P.O. Box 8000
Englewood Cliffs New Jersey 07632(US)**

(72) Inventor: **de Troostenbergh, Jean-Claude
Cleerbeekmolen 305
B-3215 Houwaart(BE)**
Inventor: **Avalosse, Bernard Léon Henri Marie
74 rue du Colmy 74
B-5544 Agimont(BE)**
Inventor: **Mignolet, René Louis
Martelarenlaan 183
B-3200 Kessel-Lo(BE)**

(74) Representative: **Wilkinson, Stephen John et al
Stevens, Hewlett & Perkins 1 St. Augustine's
Place
Bristol BS1 4UD(GB)**

EP 0 136 803 B1

## Description

The invention relates to an industrial-scale process for the production of glycerol and/or erythritol and/or ribitol, by aerobic fermentation of a sugar with a sugar-tolerant fungus such as Moniliella tomentosa var. pollinis.

It is known that aerobic fermentation of a suitable sugar by the yeast-like fungus Moniliella tomentosa var. pollinis will produce erythritol; this was first reported by G.J. Hajny, J.H. Smith and J.C. Garver in Applied Microbiology 12, pp 240-246 (May 1964), who designated the microbe as Torula $I_2A$. Later, in Antonie van Leeuwenhoek 37, pp 107-118 (1971), L. Dooms, G.L. Hennebert and H. Verachtert classified the fungus as Moniliella tomentosa var. pollinis, and confirmed its ability to produce erythritol; the authors also set forth a complete morphological description of the microbe.

In addition, they recognized that Moniliella can exist under two forms, yeast- and mould-like forms, according, essentially, to the applied culture conditions; on page 110 they state, "On agar slants both forms are produced. In stationary liquid media the mould-like form with abundant mycelium and more arthrospores than blastospores develops.

In shake-flask cultures, the yeast-like form predominates with rounded, oval and rectangular cells produced by budding and by fission while no mycelium is formed."

In attempting to design an industrial-scale process for erythritol production, employing at the start of the work, a 2-liter fermentor, and subsequently a 600-liter fermentor, problems were encountered with the distribution of the cells in the fermentation medium. It was found that the cells tended to congregate at the surface of the medium so that significant losses occurred via the foam which arises in most fermentation processes. The problem was so acute that the cells were carried out of the fermentor by the foam, and the fermentor was completely depleted of the major proportion of the cells within a short period of time. One conventional approach to the solution of the problem was to reduce the foam using a commercially available antifoam agent, and, during our investigations, a number of such products were tried, including the following : SAG 471 from Union Carbide (dimethylpolysiloxane plus silicon oxide), Biospumex 05 from Sophos (polyoxyethylene derivative in fatty alcohols), Struktol SB 2020 from Schill and Seilacher (mixture of fatty alcohols and esters), Silicon M 30 from Dow Corning Serva (30% aqueous solution of dimethylpolysiloxane) and Antifoam C from Sigma (silicone polymer). In no case was there any significant alleviation of the problem, the foam being stabilised by the microbial cells to such an extent as to make the antifoam ineffective.

We have now found that the cells are retained in the fermentation medium despite the formation of foam, by the addition to the fermentation medium of a polymer possessing certain characteristics as hereinafter described.

According to the invention, an industrial process for the production of glycerol and/or erythritol and/or ribitol, by aerobic fermentation of a suitable sugar by cells of Moniliella tomentosa var. pollinis is characterised by providing for the presence in the fermentation medium of a water-soluble or water-dispersible polymer which is capable of retaining the cells in the fermentation medium.

The polymer should be non-toxic to the Moniliella tomentosa var. pollinis and should not have an adverse effect on the fermentation process. Without wishing to be bound by any theoretical explanation, we believe that the cells of the organism form a loose association with the polymer which retains them in the fermentation broth without affecting their ability to promote the fermentation.

We have found that a particularly suitable polymer is the polysaccharide Xanthan gum although analogous polymers may be chosen by carrying out a simple test in the fermentation medium. It is possible that the association between the polymer and the cells of the organism is assisted by the presence of acidic groups on the polymer since Xanthan gum is an acidic polysaccharide.

In working with Xanthan gum, we have found that preferably at least 100 ppm is present and excellent results are obtained with about 300 ppm. More polymer may be used than is necessary for retaining the cells in the fermentation medium (i.e. more than about 500 ppm) but such excess is wasteful.

The overall process is also improved by including in the fermentation medium a conventional antifoam agent. Synthetic antifoam agents (e.g. silicone type or fatty alcohols) are preferred over the natural (e.g. lard oil) products because they can be used in smaller quantities and the final fermentation broth does not need extensive refining for their removal. With most synthetic commercial antifoam agents, 200-300 or 400 ppm is sufficient for optimum foam control.

The desired products of the process according to the present invention are erythritol and/or ribitol. Prior art workers have reported production of glycerol and arabitol, in addition to erythritol, from the fermentation of sugars by Moniliella tomentosa var. pollinis. In accordance with our process, no arabitol is produced, but ribitol, in addition to erythritol and glycerol is obtained, these three being the only polyols formed. Typically,

2

the fermentation produces a product comprising ribitol, glycerol and erythritol in the following proportions, based on total polyols: ribitol 1%-20%; glycerol 5%-40%; the balance being erythritol.

Moniliella tomentosa var. pollinis is available at the Centraal Bureau voor Schimmelcultures (CBS), Baarn, The Netherlands, as CBS 461.67, and was also deposited and is available to the public at the Commonwealth Mycological Institute Culture Collection (CMI cc), Ferry Lane, Kew, Richmond, Surrey TW9 3AF, United Kingdom, under the number (CMI cc) 271648.

The microbial cells are cultivated on a solid medium containing malt extract (4%), yeast extract (0.2%) and agar (2%). The culture so formed is then inoculated to a sterilised medium containing sugar (the amount will be discussed later) and a nitrogen source. A polymer as hereinbefore described (preferably Xanthan gum in an amount of about 300 ppm) is added, and preferably also a conventional antifoam agent (such as 200 ppm dimethylpolysiloxane antifoam). Throughout the specification and claims, all percentages and parts are by volume, unless otherwise stated.

Air is supplied to the fermentor at a rate which depends on the size of the fermentation vessel and the degree of agitation of the contents of the vessel, but is generally in the range 0.1 to 1.5 litre air/litre of fermentation medium/minute. For example, in a 2-liter vessel, the air flow rate was 1 liter/liter/minute at a stirrer speed of 400 to 800 rpm, while in a 600 liter vessel, the rate was 0.5 to 1.0 liter/liter/minute without agitation other than that produced by the air flow. The fermentation is conducted at a temperature of between 27°c and 32°C at a starting pH of between 3 and 6, preferably 4 to 5. The fermentation is stopped when the sugar has been consumed. The amount of sugar contained in the medium can be between 20% and 45%, preferably 30% to 35%. Suitable sugars for use in both the culture and the fermentation are dextrose (glucose), sucrose, fructose or maltose.

In the prior art process, as described by Hajny, various sources of nitrogen were described for use in the fermentation, for example, yeast extract, urea, corn steep liquor, malt sprouts, black strap molasses, malt extract and distillers dry solubles. In the process of the present invention good results are achieved with 0.5% yeast extract plus 0.1% urea or with 2% corn steep liquor plus 0.02% urea.

The starting pH should be between about 3.0 and 6.0 which decreases to about 2.0 to 3.5 during the fermentation. According to L. Hanssens, A. Van Regenmortel and H. Verachtert, Applied Microbiology, Vol. 24, No. 5, pp 831-833 (November 1972), laboratory fermentations held at different constant pH's result in substantial differences in yields of total polyols and erythritol. We have found that, when working with larger scale fermentations (2-liter fermentors or larger) the total polyol and erythritol yields are less sensitive to starting pH within the 4.0 to 6.0 range. In order to avoid or minimise problems of contamination, a starting pH of 4 to 5 is preferred. The fermentation is conducted until all of the sugar has been consumed (fermentation times will normally be between 4 and 12 days, depending upon the amount of sugar used), after which the culture is stopped and the cells are removed from the culture broth, e.g. by centrifuging. The cell-free culture broth, which contains erythritol, ribitol and glycerol, can be used as such, with or without refining (e.g. by ultrafiltration and demineralisation), for certain applications, e.g. in the polymer industry. The refined culture broth can also be concentrated to 60% to 80% dissolved solids and the erythritol crystallised therefrom, e.g. by the technique described by J.M. Roxburg, J.F.T. Spencer and H.R. Sallens, Canadian Journal of Technology, Vol. 34 pp 248-253 (1956). The liquor remaining after recovery of the erythritol crystals, which is also a mixture of (non-crystallised) erythritol, ribitol and glycerol, can also be used after appropriate treatment.

The following examples are intended to illustrate the practice of the invention.

Preparation of the seed culture

500 ml erlenmeyers containing 50 ml culture medium comprising 20% dextrose, 0.5% yeast extract, 0.1% urea and 300 ppm Xanthan gum were inoculated with a colony from a solid medium and cultivation was conducted for 3 to 4 days at a starting pH of 5, a temperature of 30°C and under reciprocating agitation of 100 cpm. These cultures were used in the subsequent examples.

Example 1

Two 2-liter fermentors containing 1.5 liter culture medium comprising 32% dextrose, 0.5% yeast extract and 0.1% urea were inoculated with the seed culture. To one fermentor 300 ppm of a commercially available dimethylpolysiloxane antifoam agent (SAG 471 from Union Carbide) was added; to the other 300 ppm of the antifoam plus 300 ppm Xanthan gum were added. The culture medium was supplied with air at a rate of 1 litre air/litre fermentation medium/minute and at an impeller speed of 680 rpm. The temperature was 30°C and the starting pH was 5. After two days of operation, microbial cells appeared at the surface of the broth in the fermentor to which no Xanthan gum had been added and finally were carried out of the fermentor. In the fermentor to which the Xanthan gum had been added, the cells were all retained in the broth. After 9 days, the cell density in the culture medium was $30 \times 10^6$/ml in the absence of Xanthan gum and $250 \times 10^6$ ml in the presence of Xanthan gum. The dextrose concentrations were 4% and 18% in the presence and absence of Xanthan gum, respectively, and the respective erythritol (and total polyol) concentrations were 9.2% (10.8%) and 1.5% (2.6%).

The example was repeated using the following additional commercial antifoam agents, all in an amount of 300 ppm : Biospumex 05 (Sophos), Struktol SB 2020 (Schill and Seilacher), Silicon M 30 (Dow Corning Serva). The results in each case were the same, the antifoam alone being ineffective to prevent cell depletion.

Example 2

Six 2-liter fermentors containing 1.5 liter culture medium comprising dextrose in amounts varying from 20% to 45%, 0.5% yeast extract and 0.1% urea, plus 300 ppm SAG 471 antifoam and 300 ppm Xanthan gum were inoculated as in the previous example. The impeller speed was 700 rpm, the air rate 1 liter/liter fermentation medium/ minute and the starting pH was 5. The dextrose and polyol concentrations of the culture broths were measured each day, and the cultures were allowed to ferment until the dextrose had been consumed, but for a maximum time period of nine days. The results are shown in Table 1 below :

Table 1

| Starting dextrose | Erythritol conc. % (w/v) | Total polyol( 1) % (w/v) | Erythritol yield % |
|---|---|---|---|
| 20 | 7.8 | 8.0 | 38 |
| 25 | 10.5 | 11.0 | 42 |
| 30 | 11.2 | 12.2 | 37 |
| 35 | 11.5 | 13.0 | 33 |
| 40 | 8.2 | 9.2 | 27 |
| 45 | 7.5 | 9.2 | 26 |

[1]Total polyol is erythritol and glycerol and ribitol.

As will be noted from the data, the highest erythritol concentrations were obtained with 35% dextrose, while the highest erythritol yields, based on the dextrose used, were obtained with 25% dextrose.

Example 3

Effect of varying the starting pH

2-liter fermentors containing 1.5 liter culture medium comprising 32% dextrose plus yeast extract, urea, antifoam and Xanthan gum, as in the previous example, were inoculated. The impeller speed was 680 rpm and the air rate 1 liter/liter fermentation medium/minute. The starting pH's of the culture broths were adjusted with IN HCl or IN NaOH to values of 2,3,3.5,4 and 6 respectively. The final erythritol and total polyol concentrations were measured after 9 days. The results are set forth in Table 2 :

Table 2

| Starting pH | Final pH | Erythritol concentr. % (w/v) | Total polyols concentr. % (w/v) |
|---|---|---|---|
| 6 | 2.9 | 11.1 | 14.7 |
| 4 | 2.6 | 11.7 | 13.8 |
| 3.5 | 2.2 | 9.7 | 11.4 |
| 3 | 2.0 | 7.6 | 10.0 |
| 2 | 1.9 | 0.02 | 0.02 |

It will be noted from the data that no significant differences in erythritol production, or total polyol production, resulted from the different starting pH values within the range 4 to 6.

Example 4

Effect of varying the oxygen transfer rate

Six 2-liter fermentors having culture media, Xanthan gum and antifoam identical to that of the previous example were inoculated. The air rate was 1 liter/liter fermentation medium/minute and the impeller speed rate was varied between 400 and 790 rpm. The erythritol concentrations and total polyol concentrations in the culture broths were measured after 11 days. The results are tabulated in Table 3.

Table 3

| Impeller speed | Erythritol conc. % | Total polyols conc. % |
|---|---|---|
| 400 | 8.1 | 9.3 |
| 460 | 8.1 | 9.1 |
| 585 | 8.9 | 10.1 |
| 680 | 10.7 | 13.1 |
| 740 | 11.2 | 15.9 |
| 790 | 9.2 | 16.2 |

As can be seen from the data, both the erythritol and total polyol concentrations increased with increasing aeration rate.

Example 5

Use of corn steep liquor

A 2-liter fermentor containing 1.5 liter of a culture medium comprising 32% dextrose, 2% corn steep liquor (50% dissolved solids), 0.02% urea, 300 ppm SAG 471 antifoam and 300 ppm Xanthan gum was inoculated with the seed culture. The air flow rate was 1 liter air/liter fermentation medium/minute and the impeller speed 740 rpm. The fermentation was allowed to proceed for 11 days after which time the erythritol content of the fermentation medium was found to be 11% and the total polyol content 15%. The average daily dextrose consumption was 30g/liter/day.

Example 6

A 600 liter tower-fermentation vessel containing 450 liters of a culture medium comprising 32% dextrose, 0.5% yeast extract, 0.1% urea, 300 ppm Xanthan gum and 300 ppm antifoam SAG 471 was inoculated with 5% of a seed culture. The starting pH was 5 and the air flow rate through the tower was 225 liters per minute. After 13 days the final culture broth contained 10.8% erythritol, 5.6% glycerol, 1.7% ribitol and 0.8% dextrose The erythritol yield was 34% and the total polyol yield was 56% of the consumed dextrose.

In a typical recovery operation cells were removed by centrifugation and the medium clarified by ultra-filtration and refined with ion exchangers. The colourless broth was concentrated to 70% dissolved solids and erythritol was crystallised from the liquor in the following manner. The temperature was brought to 65°C and then cooled, at a rate of 2°C per hour, to about room temperature. The crystals were recovered by filtration and washed once with ethanol. A typical crystallization yield was 74% and the composition of the liquor remaining was erythritol, 132 g/l; glycerol, 152 g/l; ribitol, 79 g/l. In general, the composition of the liquor remaining after crystallization of the erythritol may contain ribitol, glycerol and erythritol in the following proportions based on total polyols : ribitol, 5%-30%; glycerol, 30%-60%; the balance being erythritol.

**Claims**

1. An industrial process for the production of glycerol and/or erythritol and/or ribitol, by the aerobic fermentation of a suitable sugar by Moniliella tomentosa var. pollinis characterised by the provision for

5

EP 0 136 803 B1

the presence in the fermentation medium of a water-soluble or water-dispersible polymer which is capable of retaining the cells in the fermentation medium.

2. The process of Claim 1, characterised in that the polymer is Xanthan gum.

3. The process of Claim 2 characterised in that the Xanthan gum is present in an amount between 100 ppm and 500 ppm of the fermentation medium.

4. The process of Claims 1 or 2 characterised in that a conventional antifoam agent is also added to the fermentation.

5. The process of any one of the preceding claims characterised in that the air flow rate is between 0.1 and 1.5 liter/liter fermentation medium/minute.

6. The process of any one of the preceding claims characterised in that the sugar is dextrose.

7. The process of any one of the preceding claims characterised in that the sugar is present in the fermentation broth at the beginning of the fermentation in an amount of between 20% and 45%.

8. The process of any one of the preceding claims characterised in that the pH at the beginning of the fermentation is adjusted to between 3 and 6, preferably between 4 and 5.

**Revendications**

1. Procédé industriel de production de glycérol et/ou d'érythritol et/ou de ribitol, par fermentation aérobie d'un sucre convenable par Moniliella tomentosa var. pollinis, caractérisé par le fait qu'on prévoit dans le milieu de fermentation la présence d'un polymère soluble ou dispersible dans l'eau qui est capable de retenir les cellules dans le milieu de fermentation.

2. Procédé suivant la revendication 1, caractérisé en ce que le polymère est la gomme xanthane.

3. Procédé suivant la revendication 2, caractérisé en ce que la gomme xanthane est présente en une quantité comprise entre 100 ppm et 500 ppm du milieu de fermentation.

4. Procédé suivant la revendication 1 ou 2, caractérisé en ce qu'un agent antimoussant classique est aussi ajouté au milieu de fermentation.

5. Procédé suivant l'une quelconque des revendications précédentes, caractérisé en ce que la vitesse d'écoulement de l'air se situe entre 0,1 et 1,5 litre/litre de milieu de fermentation/minute.

6. Procédé suivant l'une quelconque des revendications précédentes, caractérisé en ce que le sucre est le dextrose.

7. Procédé suivant l'une quelconque des revendications précédentes, caractérisé en ce que le sucre est présent dans le bouillon de fermentation au début de la fermentation en une quantité comprise entre 20 % et 45 %.

8. Procédé suivant l'une quelconque des revendications précédentes, caractérisé en ce que le pH au début de la fermentation est ajusté entre 3 et 6, de préférence entre 4 et 5.

**Patentansprüche**

1. Verfahren zur Herstellung von Glycerin und/oder Erythrit und/oder Ribit in industriellem Umfang durch aerobe Gärung eines geeigneten Zuckers mit Moniliella tomentosa var. pollinis, dadurch gekennzeichnet, daß man Vorsorge trifft, daß im Gärmedium ein wasserlösliches oder wasserdispergierbares Polymer vorhanden ist, das in der Lage ist, die Zellen im Gärmedium zurückzuhalten.

2. Verfahren nach Anspruch 1, dadurch gekennzeichnet, daß das Polymer Xanthangummi ist.

6

3. Verfahren nach Anspruch 2, dadurch gekennzeichnet, daß der Xanthangummi in einer Menge von 100 ppm bis 500 ppm des Gärmediums vorhanden ist..

4. Verfahren nach Anspruch 1 oder 2, dadurch gekennzeichnet, daß auch ein übliches Antischaummittel zur Gärung zugesetzt wird.

5. Verfahren nach einem der vorhergehenden Ansprüche, dadurch gekennzeichnet, daß der Luftdurchsatz zwischen 0.1 und 1.5 Liter/Liter Gärmedium/Minute liegt.

6. Verfahren nach einen der vorhergehenden Ansprüche, dadurch gekennzeichnet, daß der Zucker Dextrose ist.

7. Verfahren nach einem der vorhergehenden Ansprüche, dadurch gekennzeichnet, daß der Zucker am Anfang der Gärung in der Gärbrühe in einer Menge zwischen 20% und 45% vorhanden ist.

8. Verfahren nach einem der vorhergehenden Ansprüche, dadurch gekennzeichnet, daß der pH am Anfang der Gärung auf 3 bis 6, vorzugsweise 4 bis 5, eingestellt wird.